(19)

Europäisches Patentamt

European Patent Office

Office européen des brevets

(11) **EP 2 799 866 A1**

(12) **EUROPEAN PATENT APPLICATION**

(43) Date of publication:
**05.11.2014 Bulletin 2014/45**

(51) Int Cl.:
**G01N 33/18** (2006.01)     **G01N 21/64** (2006.01)

(21) Application number: **13305580.6**

(22) Date of filing: **02.05.2013**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicant: **Université Paris-Est Marne-la-Vallée 77420 Champ-sur-Marne (FR)**

(72) Inventors:
• **Oturan, Mehmet A.**
  **94350 Villiers sur Marne (FR)**

• **Mousset, Emmanuel**
  **17600 Saujon (FR)**
• **Van Hullebusch, Eric**
  **91250 Tigery (FR)**
• **Oturan, Nihal**
  **94350 Villiers sur Marne (FR)**

(74) Representative: **Regimbeau**
  **20, rue de Chazelles**
  **75847 Paris Cedex 17 (FR)**

(54) **Method for determining the concentration of non-ionic surfactants in an aqueous solution by excitation-emission fluorescence spectroscopy based on the enhancement of the fluorescence of 6-p-toluidino-2-naphthalenesulfonic acid**

(57) The present invention relates to a method for determining the concentration of a nonionic surfactant in an aqueous solution by excitation-emission fluorescence spectroscopy based on the enhancement of the fluorescence of 6-p-toluidino-2-naphthalenesulfonic acid (TNS), the use of said method for optimizing the consumption of a surfactant in a wastewater treatment process and a soil remediation process comprising said method.

EP 2 799 866 A1

**Description**

The present invention relates to a method for determining the concentration of a non-ionic surfactant in an aqueous solution by excitation-emission fluorescence spectroscopy based on the enhancement of the fluorescence of 6-p-toluidino-2-naphthalenesulfonic acid (TNS), the use of said method for optimizing the consumption of a non-ionic surfactant in a waste-water treatment process and a soil remediation process comprising said method.

Surface-active agents or "surfactants" are amphiphilic molecules having both a hydrophobic (apolar group) tail and a hydrophilic (polar group) head. When dissolved in water at low concentrations, surfactant molecules exist as monomers. When the concentration of surfactant increases, there is a critical concentration beyond which surfactant monomers start aggregating to form self-assemblies called micelles. The concentration at which micelle formation occurs is known as the critical micelle concentration (CMC). CMC is a function of surfactant structure, composition of the solution, temperature, ionic strength, and the presence and types of organic additives in the solution.

Depending on the nature of the hydrophilic group, surfactants can be classified as anionic, cationic, zwitterionic and non-ionic.

Surfactants have several applications not only in soap and detergent industry but also in medicine, and as extracting agents in chemistry and in environmental technology, especially in soil and groundwater remediation.

In surfactant-enhanced remediation of contaminated soil, anionic and non-ionic surfactants are mostly used especially to extract hydrophobic organic contaminants since they are strongly sorbed to soil. These pollutants are also known to be persistent in the environment and have potential toxicity effect.

Among the non-ionic surfactants, which are better solubilizing agents than anionic and cationic ones because of their lower CMC value, their lower sorption into soil and their better cost-effectiveness, polysorbates are widely used. In particular, polyoxyethylene (20) sorbitan monooleate, sold under the trade name Tween 80® is getting more and more interesting since it can enhance also phytoremediation of contaminated soils. A more recent study shows the potential benefit of Tween 80® in contaminated soil bioremediation by enhancing the interaction between organic pollutants and bacteria.

It is very interesting to quantify the surfactant evolution during a soil remediation process, in particular, its sorption into soil and its degradation during a bioremediation process or a water treatment of soil washing/flushing solution containing such surfactant. Its ability to be reused during a soil washing/flushing treatment can then be studied.

Several analytical methods already exist to quantify general surfactants such as gas chromatography, gravimetric method, flow-injection methods and dynamic surface tension detection. These methods are based on the liquid-liquid extraction but have low sensitivity and selectivity.

Few techniques have been developed so far to quantify non-ionic surfactants and especially Tween 80®. Among them, colorimetric measurements, cobalt thiocyanate active substances method and potassium picrate active substances method, direct UV absorbance at a wavelength of 234 nm and total organic carbon (TOC).

High performance liquid chromatography method with derivatization of stationary phase was also experimented using a complexing agent such as phenyl isocyanate to produce a UV active derivative upon reaction with the ethoxylate group. However, at low concentrations (below 0.6 g $L^{-1}$), the accuracy of measurement was unacceptable.

One other method was developed to quantify Tween 60® surfactant based on fluorescence enhancement of tetraphenylporphyrin. However, this method is not selective and efficient enough when it is applied for the quantification of other surfactants, in particular Tween 80®.

The methods available so far are hence not satisfying, especially when studying solutions containing other organic molecules and there exists a need for a quantification method of surfactants, in particular in aqueous solutions containing other molecules such as organic pollutants, organic matter or oxidation by-products that can absorb in the same range of wavelength and whose carbon are also taken into account in TOC values.

As is well known by the skilled person, a fluorescent molecule is capable of absorbing energy at a defined wavelength called *excitation wavelength* and to release this energy at another wavelength called *emission wavelength.* The *excitation wavelength* and the *emission wavelength* are specific to the fluorescent molecule and will depend on the type of liquid medium in which the surfactant is present.

6-p-toluidino-2-naphthalenesulfonic acid (TNS) is a molecule that becomes fluorescent upon complexation and has already been used for the quantification of cyclodextrins, a host/guest molecule, by fluorescence. However those experiments allowed only a rough assessment of cyclodextrins concentration compared to chromatographic methods, since slightly modified cylcodextrins are also detected.

The inventors have discovered that while non-ionic surfactants have no fluorescence in the absence of 6-p-toluidino-2-naphthalenesulfonic acid and the fluorescence of TNS alone is negligible, the formation of micelles between a non-ionic surfactant and TNS led to an enhancement of the fluorescence of TNS.

This fluorescence enhancement can be unexpectedly taken advantage of for determining the concentration of a non-ionic surfactant in an aqueous solution containing other organic molecules.

It is therefore an object of the present invention to provide an improved method for determining the concentration of non-ionic surfactant in an aqueous solution, in particular in an aqueous solution containing other molecules.

It is another object of the present invention to provide a method for monitoring the concentration of non-ionic surfactant in an aqueous solution during a wastewater treatment process, in particular during a soil remediation process.

It is a further object of the present invention to provide a soil remediation process comprising a step of determining the concentration of non-ionic surfactants in the aqueous solution used for washing a polluted soil.

An advantage of the method according to the present invention over the ones described in prior art is the significant improvement of the detection limit and the quantification limit allowing the accurate determination of the concentration of non-ionic surfactants in an aqueous solution.

A particular advantage of the present method is that the concentration of non-ionic surfactants in the aqueous solution can be accurately determined in the presence of organic matter. Such organic molecules include pollutants and humic acid like substances, which can represent between 70% to 90% by weight of the soil organic matter and show an excitation range comprised between 250-360 nm and an emission range comprised between 380-480 nm. The presence of these organic molecules has led to the low performance of UV absorbance and TOC analysis used in prior art.

In a first embodiment according to the present invention, a method for determining the concentration of a non-ionic surfactant in an aqueous solution by excitation-emission fluorescence spectroscopy based on the enhancement of the fluorescence of 6-p-toluidino-2-naphthalenesulfonic acid (also referred to as TNS in the present application) upon formation of a surfactant-TNS complex is provided.

The method comprises the steps of:

(a) contacting a sample of an aqueous solution containing non-ionic surfactant with TNS,
(b) measuring the emission fluorescence of the sample of said aqueous solution,
(c) deducing the concentration of non-ionic surfactant in the sample from the emission fluorescence of said aqueous solution.

The method is useful for determining the concentration of non-ionic surfactants in any aqueous solution but can preferably be used with aqueous solutions containing other organic molecules, even more preferably aqueous solutions containing organic pollutants, such as soil and groundwater remediation waters, landfill leachates and industrial wastewater containing for example saturated hydrocarbons, polycyclic aromatic hydrocarbons, polychlorinated biphenyl, volatile organic compounds, pharmaceutical substances or dyes.

According to the present invention, the term "surfactant" means a molecule having a hydrophobic tail and a hydrophilic head. In the sense of the present invention, cyclodextrins and their derivatives are not considered as surfactants. In the present invention, the method is used with non-ionic surfactants. Non-ionic surfactants have no charge groups on their hydrophilic head. Non-ionic surfactants include but are not limited to Polysorbates such as Polysorbate 20 (Tween 20®, Polyoxyethylene (20) sorbitan monolaurate), Polysorbate 40 (Tween 40®, Polyoxyethylene (20) sorbitan monopalmitate), Polysorbate 60 (Tween 60®, Polyoxyethylene (20) sorbitan monostearate), Polysorbate 80 (Tween 80®, Polyoxyethylene (20) sorbitan monooleate) or Polyethylene glycol tert-octylphenyl ether (Triton® X100), polyoxyethylene (20) cetyl ether (Brij 58®), polyoxyethylene (4) lauryl ether (Brij 30®), polyoxyethylene (23) lauryl ether (Brij 35®), octylphenol polyoxyethylene (Igepal CA-720), nonyl phenyl polyoxyethylene glycol (Tergitol NP-10).

In step (a), a sample of the aqueous solution containing the non-ionic surfactant is taken out and contacted with TNS. The sample can either be added to the appropriate amount of solid TNS, added to the appropriate amount of a solution containing TNS or TNS can be added to the sample as a solid or in solution. In a preferred embodiment, the sample is diluted or concentrated before the addition of TNS in order to reach the surfactant concentration range of interest. In a more preferred embodiment, the sample is diluted before the addition of TNS in order to reduce fluorescence interference and reach the surfactant concentration range of interest.

The concentration of TNS in the sample is comprised between $10^{-6}$ M and $10^{-4}$ M, preferably between $10^{-5}$ M and $10^{-4}$ M. Advantageously, the concentration of 6-p-toluidino-2-naphthalenesulfonic acid is comprised between $10^{-5}$ M and $9 \times 10^{-5}$ M, a concentration of about $5 \times 10^{-5}$ M being especially preferred.

The appropriate concentration may vary, depending on the quantity and the nature of the surfactant present in the solution and the sensitivity of the spectrofluorometer. It is obvious to the skilled person to adjust these concentrations by mere routine experiments.

In step (b), the emission fluorescence of the solution is measured.

The fluorescence emission can be measured by methods well known in the art for this purpose. Particularly considered are spectrofluorometers of all types. Advantageously, the spectrofluorometer can be used on the whole wavelength spectrum (200-800 nm) both for excitation and emission.

The fluorescence detection can also be performed with an HPLC apparatus coupled with a fluorescence detector. The aqueous solution is diluted to a dilution ratio suitable for HPLC analysis and the sample is injected in the HPLC apparatus.

For measuring the emission fluorescence of the surfactant-TNS complex, TNS is mixed with the eluent used for the separation. This method allows separating the non-ionic surfactant from the other organic molecules and provides a more selective method for the detection of the emission fluorescence of the surfactant-TNS complex.

The excitation wavelength and the emission wavelength are dependent of the non-ionic surfactant studied. Optimal values can be easily determined by the skilled person by scanning different excitation and emission wavelengths with the spectrofluorometer.

For the non-ionic surfactant-TNS complex, the optimal excitation wavelength is typically comprised between 200 and 400 nm, preferably between 300 and 350 nm and even more preferably between 310 and 320 nm.

For the non-ionic surfactant--TNS complex, the optimal emission wavelength is typically comprised between 300 and 500 nm, preferably between 400 and 450 nm and even more preferably between 425 and 435 nm.

For the non-ionic surfactant-TNS complex, the preferred excitation wavelength is 318 nm and the preferred emission wavelength is 428 nm.

The intensity of the emission fluorescence is the value used to determine the concentration of the non-ionic surfactant in the sample of the aqueous solution. This value can be read directly on the spectrofluorometer.

In step (c), the intensity of the emission fluorescence of the sample measured in step (b) is correlated with the concentration of non-ionic surfactant in the sample.

It is first necessary to establish the relation between the intensity of the emission fluorescence of the surfactant/TNS complex and the concentration of non-ionic surfactant in water (without TNS). The emission fluorescence intensity being dependent of the complexation constant of a non-ionic surfactant, the emission fluorescence intensity will depend on the surfactant.

In a first alternative, the relation between the concentration of the non-ionic surfactant and the emission fluorescence intensity is established using the partitioning model of the organic compounds between micelles and monomeric solution, which quantify the surfactant solubilization.

The micelle phase/aqueous phase partition coefficient ($K_{mw}$) is based on the mole fraction ratios, *i.e.* the ratio of mole fraction of the compound in the micellar pseudophase ($X_m$) to the mole fraction of the compound in the aqueous pseudophase ($X_a$). $K_{mw}$ also can be defined as in equation (1):

$$K_{mw} = \frac{X_m}{X_a} = \frac{C_m}{C_a} = \frac{S - S_{CMC}}{(C_S - CMC + S - S_{CMC})(S_{CMC}V_W)} \qquad (1)$$

where $C_m$ is the concentration of the hydrophobic solute in the micelle, $C_a$ is the concentration in the aqueous phase, CMC is the critical micelle concentration, S is the apparent solubility of organic compound at surfactant concentration $C_S$ ($C_S > CMC$), $S_{CMC}$ is the apparent solubility of the organic compound at the CMC, $V_w$ is the molar volume of water, *i.e.,* $1.805 \times 10^{-3}$ L mol$^{-1}$ at 22°C.

As the concentration of surfactant and TNS are low, the following equations (2) and (3) can be written:

$$F = kI_0 S \qquad (2)$$

$$F_0 = kI_0 S_{CMC} \qquad (3)$$

where F and $F_0$ are the emission fluorescence referred to S and $S_{CMC}$ respectively, k is a constant (depending on the equipment, the compounds studied, the molar absorption coefficient and the length of the container) and can be determined by the skilled person at a given concentration by using the slope of the curve obtained with equation (4), all other values being known ($K_{mw}$, $I_0$, $F_0$, Vw) and $I_0$ is the radiation intensity of excitation. The fluorescence of the surfactant alone is considered to be equal to zero.

By replacing equations (2) and (3) in equation (1) the following linear equation (4) between F and $C_s$ is obtained:

$$F = a \times C_S + b \qquad (4)$$

$$\text{with } a = \frac{F_0 V_W K_{mw} kI_0}{kI_0 - K_{mw} F_0 V_W} \text{ and } b = F_0 - \frac{F_0 V_W K_{mw} kI_0}{kI_0 - K_{mw} F_0 V_W} CMC$$

By replacing the variables in equation (4) by the appropriate values, the emission fluorescence intensity measured in step (b) can be correlated to the concentration of the surfactant in solution and the concentration of non-ionic surfactant can be determined.

In a second alternative, a calibration curve is used.

The calibration curve is a well-known method for determining the concentration of a substance in an unknown sample by comparing it to a set of standard samples of known concentrations.

The calibration curve is established by plotting the emission fluorescence intensity of aqueous solutions containing known concentrations of a non-ionic surfactant and a fixed concentration of TNS as a function of the concentration of said non-ionic surfactant.

The standard samples are preferably prepared in water, even more preferably in ultra-pure water.

It is manifest that the concentration of TNS in the standard samples should be the same as the concentration of TNS in the unknown sample for correlation purposes.

The emission fluorescence intensity of the unknown sample measured in step (b) is then compared with the calibration curve to determine the concentration of the non-ionic surfactant in the sample.

In one advantageous embodiment according to this first embodiment, the quantification method is used for the determination of the concentration of Tween 80® in an aqueous solution, in particular in an aqueous solution containing pollutants or humic acid like substances.

In that case, the emission fluorescence intensity of the Tween 80®-TNS complex in the sample of the aqueous solution is measured at an excitation wavelength of about 318 nm and at an emission wavelength of about 428 nm with a concentration of TNS comprised between $10^{-6}$ M and $10^{-4}$ M, preferably at a concentration of about $5 \times 10^{-5}$ M.

The concentration of Tween 80® in the sample is then determined by using equation (4) or by comparison with a calibration curve, obtained by plotting the emission fluorescence intensity of the Tween 80®-TNS complex measured at an excitation wavelength of about 318 nm and at an emission wavelength of about 428 nm with a concentration of TNS comprised between $10^{-6}$ M and $10^{-4}$ M, preferably at a concentration of about $5 \times 10^{-5}$ M as a function of the Tween 80® concentration.

The method therefore comprises the steps of:

(a) Contacting a sample of an aqueous solution containing Tween 80® with TNS to obtain a final TNS concentration of about $5 \times 10^{-5}$ M in the sample,
(b) measuring the fluorescence emission of the sample of said aqueous solution at an excitation wavelength of about 318 nm and at an emission wavelength of about 428 nm,
(c) deducing the concentration of Tween 80® in the sample from the emission fluorescence of said aqueous solution by using equation (4) or by comparing the emission fluorescence intensity measured with a calibration curve.

In a second embodiment according to the present invention, a method for monitoring the concentration of a non-ionic surfactant in an aqueous solution during a wastewater treatment process is provided.

According to the present invention, the term "wastewater treatment process" refers to processes devoted to the removal of organic pollutants from the effluent. The wastewater treatment process of the present invention are amenable to the treatment of several effluents, for example, but not limited to, soil and groundwater remediation waters, municipal wastewater, industrial wastewater or process water and landfill leachates.

From an economic standpoint, it is interesting to monitor the evolution of the concentration of a non-ionic surfactant during a wastewater treatment process for reducing the consumption of surfactant.

Determining the concentration of non-ionic surfactant in an aqueous solution at the end of a wastewater treatment process enables recycling the aqueous solution for a further extraction cycle with an adjustment, if the case may be, of the surfactant concentration.

As excessive surfactant concentrations can be detrimental in wastewater treatment process, determining the concentration of non-ionic surfactant in the aqueous solution of a wastewater treatment process is desirable. For example, in municipal wastewater treatment determining the concentration of non-ionic surfactant enables the addition of the appropriate quantity of antifoaming agents.

Aqueous solution containing non-ionic surfactants are commonly used in many extraction processes such as a soil remediation process in which contaminated soil are washed with the aqueous solution containing non-ionic surfactants.

The aqueous solution is then treated, in particular submitted to a decontamination step, for removal of the extracted contaminants.

It is interesting from an economic point of view to then recycle the decontaminated aqueous solution for a further extraction cycle. This requires a measurement of the non-ionic surfactant concentration in the decontaminated aqueous solution in order to ensure that the aqueous solution contains a sufficient amount of non-ionic surfactant.

As a non-limitative example, in a soil remediation process, the concentration of non-ionic surfactant is reduced because of its adsorption in the soil during the washing step and its degradation during the aqueous solution decontamination step. Monitoring the concentration of non-ionic surfactant after the decontamination step enables an addition of the required amount of surfactant before recycling and reusing the aqueous solution for a further cycle in just the necessary amount.

The present invention therefore also relates to a method for monitoring the concentration of non-ionic surfactants in an aqueous solution during a water treatment process, preferably after a decontamination step, comprising the determination of the concentration of said non-ionic surfactants in the aqueous solution by excitation/emission fluorescence spectroscopy with TNS as described previously.

Accordingly, the method comprises the steps of:

(a) determining the concentration of non-ionic surfactant in the aqueous solution by excitation-emission fluorescence spectroscopy as described previously,
(b) comparing the concentration of non-ionic surfactant in the aqueous solution with the required concentration, and
(c) if necessary, adjusting the concentration of non-ionic surfactant in the aqueous solution to the required concentration and recycle the aqueous solution for a further cycle, or dispose of the aqueous solution.

By "required concentration", it is meant in the sense of the present invention, the concentration of non-ionic surfactant needed to extract the pollutants in an optimal manner. This value depends on the process and the type of pollutants and can be determined by the normal technician.

The monitoring of the concentration of non-ionic surfactant can be performed during the treatment step by taking out samples at different intervals. This allows monitoring the decay of the non-ionic surfactant during the treatment.

The concentration of non-ionic surfactant in the aqueous solution can also be determined after the treatment/decontamination step.

In wastewater treatment processes where the aqueous solution is recycled for a further extraction step, the determination of the concentration of non-ionic surfactant is preferably performed before the aqueous solution is recycled in the extraction step but may also be performed directly in the reactor where the extraction of the pollutants takes place.

The non-ionic surfactant is preferably Tween 80® and step (a) is conducted using the preferred conditions described previously.

In a third embodiment according to the present invention, a soil washing process comprising a step of determining the concentration of non-ionic surfactant in a decontaminated aqueous solution with TNS is provided.

Surfactant enhanced soil remediation processes essentially consist in the extraction of the pollutants contained in a contaminated soil with an aqueous solution containing a surfactant (solid/liquid extraction). The cleaned soil and the contaminated aqueous solution are separated, for example by gravitation, decantation, centrifugation, or filtration and the aqueous solution is then treated for decontamination. The decontaminated aqueous solution may then be sent to a wastewater treatment plant for further decontamination but is preferably reused in the solid-liquid extraction step.

The soil remediation process according to the present invention advantageously comprises the steps of:

(a) washing the contaminated soil by solid-liquid extraction by contacting an aqueous solution containing a non-ionic surfactant with said soil,
(b) separating the contaminated water from the cleaned soil,
(c) treating the contaminated water,
(d) determining the concentration of non-ionic surfactant in the aqueous solution by excitation-emission fluorescence spectroscopy as described previously,
(e) if the case may be, adding non-ionic surfactant, and
(f) recycling the aqueous solution containing the non-ionic surfactant in the soil washing step (a).

The non-ionic surfactant used for washing the soil is one commonly employed in soil remediation processes. Preferably, the non-ionic surfactant is Triton® X100 or Tween 80®, even more preferably Tween 80®.

The required concentration, time and parameters for the solid-liquid extraction essentially depend on the type of soil, the type of pollutants and can be determined accordingly by the normal technician.

After the cleaned soil has been separated from the contaminated aqueous solution, the contaminated aqueous solution containing the surfactant is treated by known methods such as ozonation, photo-oxidation ($O_3$/UV, $H_2O_2$/UV or

TiO$_2$/UV), permanganate or persulfate oxidation, electrochemical treatment, bioremediation and advanced oxidation processes such as Fenton treatment (H$_2$O$_2$/Fe$^{2+}$) and electro-Fenton process.

Electro-Fenton is an emerging advanced oxidation process that consists of a coupling between electrochemistry and Fenton process since the Fenton's reagent is electrochemically *in situ* generated (Oturan 2000).

$$Fe^{2+} \,|+ H_2O_2 \rightarrow Fe^{3+} + HO^- + {}^*OH$$

It permits minimizing the use of H$_2$O$_2$ reagent that is generated *in-situ* and continuous regeneration of soluble iron (Fe$^{2+}$, Fe$^{3+}$, or iron oxides) from a catalytic amount added initially to the solution.

The soil remediation process according to the invention preferably comprises a step of electro-Fenton treatment of the contaminated aqueous solution containing the non-ionic surfactant.

In a soil washing process, iron is already present in the soil and addition of soluble iron may not be required. A catalytic amount of iron may be added depending on the water to treat.

An inert electrolyte may be added to the medium. These electrolytes are well known in the art. A preferred electrolyte is Na$_2$SO$_4$.

The cathode may be any cathode usually used in electro-Fenton, but is preferably a carbon-felt piece cathode. The anode may also be any anode usually employed in electro-Fenton, advantageously a boron doped diamond (BDD) plate anode.

Preferably, the anode is centered in the electrochemical reactor and the cathode covers the inner wall of the electrochemical reactor.

The electro-Fenton is conducted at an initial acidic pH, preferably about 3.

Oxygen may be added in the reaction solution during the electro-Fenton treatment up to the O$_2$ saturation level. The appropriate level of oxygen may be added in the form of pure oxygen or compressed air.

The concentration of non-ionic surfactant is determined with TNS in the conditions described previously. Advantageously, the concentration is determined at the end of the treatment of the contaminated water and before the aqueous solution is recycled to the solid-liquid extraction step.

If the concentration of surfactant in the treated aqueous solution is considered insufficient, more surfactant is added up to the required concentration. The surfactant can be added before the aqueous solution is recycled in the solid-liquid extraction step or directly in the compartment where the soil washing step takes place, depending on the installation used.

In a preferred embodiment according to the present invention, a soil remediation process is provided in which the non-ionic surfactant is Tween 80® comprising the steps of:

(a) washing the contaminated soil by contacting an aqueous solution containing Tween 80® with said soil,
(b) separating the contaminated water from the cleaned soil,
(c) treating the contaminated water, preferably by electro-Fenton,
(d) determining the concentration of Tween 80® in the aqueous solution as described previously,
(e) if necessary, adding Tween 80® to reach the required concentration, and
(f) recycling the aqueous solution containing Tween 80® in the soil washing step (a).

In a fourth embodiment, the invention concerns the use of 6-(p-toluidino)naphthalen-2-sulfonic acid (TNS) for determining the concentration of non-ionic surfactants, advantageously non-ionic polysorbates surfactants in an aqueous solution by excitation-emission fluorescence spectroscopy.

Advantageously, TNS is used for determining the concentration of polysorbates, Triton® X 100, more advantageously polysorbate 80 (Tween 80®).

While TNS can be used for determining the concentration of a non-ionic surfactant in any aqueous solution, it is advantageously used for aqueous solutions containing other organic molecules that may render the analysis with other methods false, such as UV-Vis spectroscopy or TOC.

In an advantageous embodiment, TNS is used for determining the concentration of a non-ionic surfactant, advantageously Tween 80®, in an aqueous solution containing pollutants or contaminants such as soil and groundwater remediation waters, municipal wastewater, industrial wastewater or process water and landfill leachates containing for example saturated hydrocarbons, polycyclic aromatic hydrocarbons, polychlorinated biphenyl, volatile organic compounds, pharmaceutical substances or dyes.

Figure 1 represents the calibration curve of Tween 80® determined by fluorescence (Excitation-Emission: 318-428nm) in the presence of TNS (5 × 10$^{-5}$ M). The X-axis represents the concentration of Tween 80® in mg. L$^{-1}$ and the Y-axis the fluorescence intensity.

Figure 2 illustrates the UV-absorbance spectra of Tween 80® (small dashes curve), phenanthrene (full line curve) and Tween 80® (long dashes curve) in the presence of phenanthrene. The X-axis represents the wavelength (in nm) and the Y-axis absorbance.

Figure 3: A represents excitation-emission matrix spectra of phenanthrene with Tween 80® in the absence of TNS and B represents excitation-emission matrix spectra of phenanthrene with Tween 80® in the presence of TNS. The X-axis represents the emission wavelength in nm and the Y-axis the excitation wavelength in nm.

Figure 4: A represents excitation-emission matrix spectra of phenanthrene and oxidation by-products with Tween 80® in the absence of TNS after 2 hours of electro-Fenton treatment and B represents excitation-emission matrix spectra of phenanthrene and oxidation by-products with Tween 80® in the presence of TNS after 2 hours of electro-Fenton treatment. The X-axis represents the emission wavelength in nm and the Y-axis the excitation wavelength in nm.

Figure 5 represents the decay of Tween 80® (▲), phenanthrene (□) and Total organic carbon (TOC, X) during electro-Fenton. Tween 80® decay is determined by the fluorescence method of the invention. The X-axis represents the decay in % and the Y-axis treatment time in minutes.

Figure 6: A represents the excitation-emission matrix spectra of soil washing solution by using Tween 80® without the addition of TNS and B represents the excitation-emission matrix spectra of soil washing solution by using Tween 80® in the presence of TNS. The X-axis represents the emission wavelength in nm and the Y-axis the excitation wavelength in nm.

## EXAMPLES

In the aim to study the possible interferences of phenanthrene, its oxidation by-products and soil organic matter, excitation-emission matrix fluorescence spectroscopy analyses were performed. The samples were first diluted with ultra-pure water at the same dilution factor to be comparable. Fluorescence spectra of the sample were measured using a Shimadzu (Japan) RF-5301 PC 170 spectrofluorophotometer. Spectra were collected with subsequent scanning of emission spectra from 220 to 550 nm by varying the excitation wavelength from 220 to 450 nm at 12 nm increments using high sensitivity. The software Panorama Fluorescence 2.1 was employed for handling excitation-emission matrix data.

## Example 1: Assessment of the fitting between the calibration curve and the concentration determined using equation (4) for Tween 80®

Fluorescence was measured on a Kontron Instruments SFM 25 spectrofluorometer, USA. Different excitation and emission wavelengths were investigated out with the spectrofluorometer and finally the highest sensitivity was obtained at 318 nm for excitation and 428 nm for emission. Each sample was diluted in TNS ($5 \times 10^{-5}$ M).

Results:

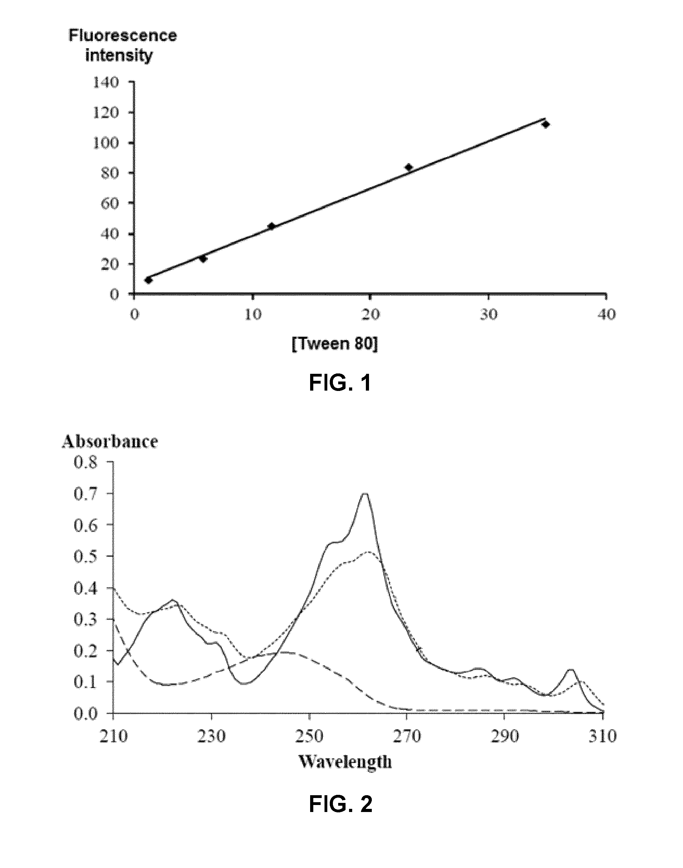

The calibration curve obtained is depicted in Figure 1.

For the Tween 80® fluorimetric analysis method with an excitation-emission wavelength of 318-428 nm, the suggested linear model between fluorescence measurement of Tween 80®-TNS and Tween 80® concentration fit well ($R^2$ = 0.995) the experimental calibration curve (F = 3.1123 ($\pm$ 0.12) $\times$ [Tween 80®] + 7.1849 ($\pm$ 2.33)).

The calibration curve is also usable for Tween 80® concentrations below the CMC (15.7 mg L$^{-1}$ (Rosas et al. 2011)) in contrast to the assumption considered in the model.

The fluorimetric method provided for Tween 80® analysis a detection limit of 0.13 mM (0.10 mg C L$^{-1}$) and a quantification limit of 0.39 mM (0.30 mg C L$^{-1}$).

Comparatively, the detection limit and the quantification limit were 3.18 mM (2.44 mg C L$^{-1}$) and 9.64 mM (7.40 mg C L$^{-1}$) respectively for UV absorption method and 0.27 mM (0.21 mg C L$^{-1}$) and 0.85 mM (0.65 mg C L$^{-1}$) respectively for TOC method.

These results clearly highlight the advantages of the fluorimetric method over the prior art methods even below the CMC, whether a calibration curve or equation (4) is employed to determine the concentration of Tween 80® in the sample.

## Example 2: Interference of phenanthrene on the Tween 80®-TNS complex UV absorption spectrum and fluorescence emission spectrum.

Phenanthrene, a hydrophobic organic contaminant representative of the polycyclic family was selected as the test compound.

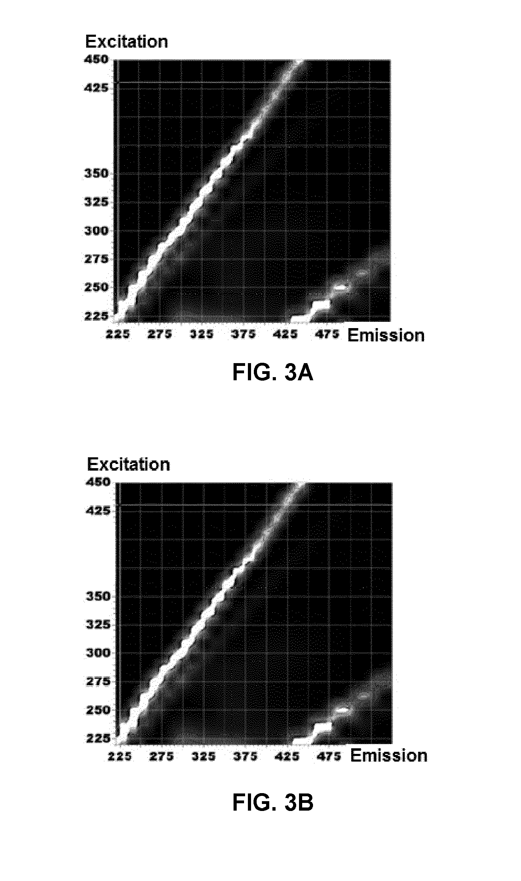

A model aqueous solution containing 2 mg L$^{-1}$ of phenanthrene and 750 mg L$^{-1}$ of Tween 80® was analyzed by UV spectroscopy (figure 2) and emission fluorescence spectroscopy in the presence (figure 3a) and in the absence (figure 3b) of 6-(p-toluidino)naphthalen-2-sulfonic acid.

Results:

UV absorbance spectra of Phenanthrene and Tween 80® overlap with each other and the selective detection of Tween 80® using UV spectroscopy is not a reliable tool for determining the concentration of Tween 80 ® when phenanthrene is present in the aqueous solution.

As shown by figure 3b, there is in contrast no impact of phenanthrene on the fluorescence of the TNS-Tween 80® complex.

Using the calibration curve of example 1 or equation (4); the concentration of Tween 80® can be determined even in the presence of phenanthrene thanks to the high sensitivity of the method.

**Example 3: Interference of phenanthrene oxidation by-products on the Tween 80®-TNS complex fluorescence emission spectrum.**

The model aqueous solution of example 2 was subjected to an electro-Fenton step.

Electro-Fenton experiment was performed at room temperature ($22 \pm 1°C$), in a 0.40 L undivided glass electrochemical reactor at current controlled conditions. The cathode was a 150 $cm^2$ carbon-felt piece (Carbone-Lorraine, France). The anode was a 5 cm height cylindrical (i.d. = 3 cm) Pt grid, which is centered in the cell and surrounded by cathode covering the inner wall of the cell. An inert electrolyte ($Na_2SO_4$ at 0.150 M) was added to the medium. Since too much foam is formed during bubbling system, the solutions containing Tween 80® were not saturated with $O_2$. The electrochemical cell is monitored by a power supply HAM EG 7042-5 (Germany) and applied current was set to 1000 mA. Solutions were stirred continuously by a magnetic stirrer. A heat exchanger system was used to keep the solution at constant room temperature by using fresh water. The pH of initial solutions was set at the optimal value of 3.0 ($\pm$ 0.1) by the addition of aqueous $H_2SO_4$ (1 M) solution. In these experiments $FeSO_4 \cdot 7H_2O$ was added at catalytic amount (0.2 mM). Tween 80® (750 mg $L^{-1}$) was used in the presence of phenanthrene in excess (17 mg $L^{-1}$ initially).

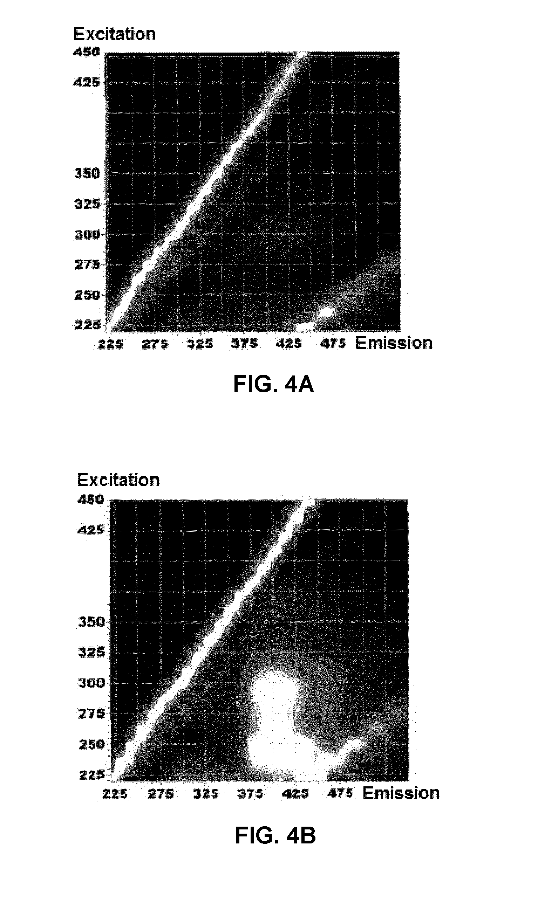

Emission fluorescence was measured in the presence (figure 4a) and in the absence (figure 4b) of TNS after 2 hours of treatment.

The phenanthrene degradation was followed by reversed phase with a high performance liquid chromatography coupled with a diode array detector from Dionex (USA). The detection was carried out at the wavelength of 249 nm. The mobile phase was a mixture of water/methanol (22:78 v/v) at the flow rate of 0.8 mL $min^{-1}$ (isocratic mode), giving a 6.9 min of retention time for phenanthrene. A reversed-phase C-18 end capped column (Purospher®, Merck, Germany) placed in an oven set at 40°C was used.

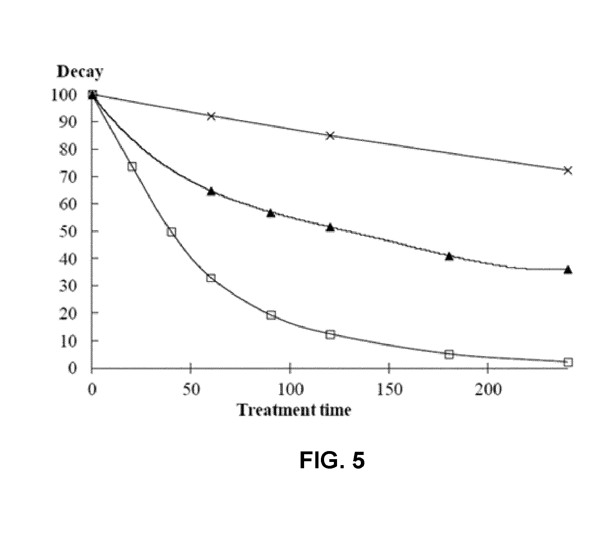

The electro-Fenton degradation of Tween 80® was measured by the method of the present invention and by TOC (Figure 5).

Results:

Electro-Fenton treatment allows the complete oxidation of Phenanthrene in about 240 minutes.

The fluorescence intensity of phenanthrene and its oxidation by-products are insignificant in this range of concentration (< 3.5%) and have no impact on the fluorescence of the TNS-Tween 80® complex.

Since TOC values take into account all the carbons present in the solution, all the Tween 80®, phenanthrene and oxidation by-products are considered, leading to a higher and incorrect value compared to fluorescence data.

Using the calibration curve of example 1 or equation (4), the concentration of Tween 80® can be determined even in the presence of phenanthrene and Tween 80® oxidation by-products thanks to the high sensitivity of the method.

**Example 4: Interference of soil organic matter on fluorescence detection**

In the aim to study the possible interferences of soil organic matter, excitation-emission matrix fluorescence spectroscopy analyses were performed.

The polluted soil was sampled from a polycyclic aromatic hydrocarbons and aliphatic hydrocarbons contaminated site. Before its utilization, the soil was sieved under 2 mm and homogenized by a sample divider (Retsch, Germany).

The soil had the following particle size distribution: clay (< 2 $\mu$m): 19.7%, fine silt (2-20 $\mu$m): 23.3%, coarse silt (20-50 $\mu$m): 7.5%, fine sand (50-200 $\mu$m): 12.3%, coarse sand (200-2000 $\mu$m): 37.1%. The soil had the other following characteristics: pH (water) = 8.3, organic matter content: 4.71 %, total polycyclic aromatic hydrocarbons (16 compounds) content: 730 mg $kg^{-1}$, aliphatic hydrocarbons ($C_{10}$-$C_{40}$) content: 850 mg $kg^{-1}$.

The soil washing experiment was performed in a 500 mL bottle at a soil/liquid ratio equal to 10% (40 g/ 400 mL). A Tween 80® solution (10 g L$^{-1}$) was used and the mixture was rotated in a Rotoshake RS12 (Gerhardt, Germany) at 10 rotations per minute for 24 h. Then the particles settled for 12 h and the supernatant was filtered with a 0.7 $\mu$m glass microfiber filter (Whatman GF/F, England). The supernatant was diluted 15,000 times, and analyzed by excitation-emission matrix fluorescence spectroscopy with or without adding TNS (1.7 × 10$^{-6}$ M).

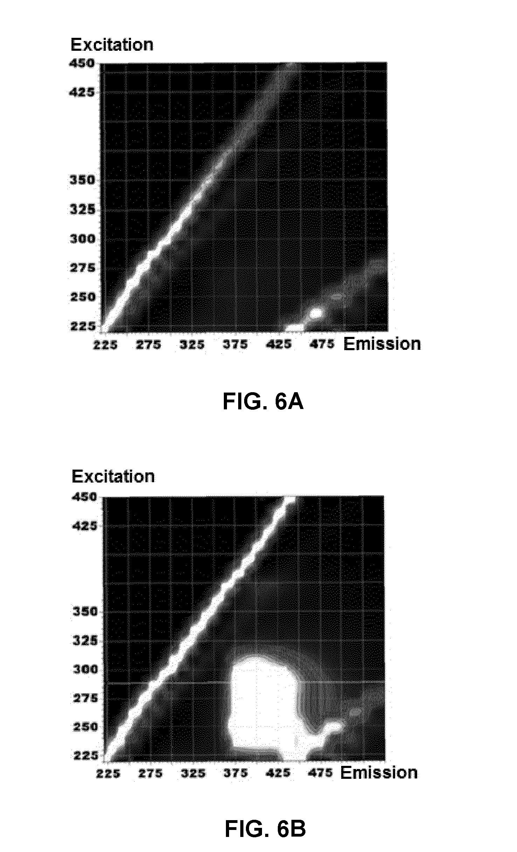

Emission fluorescence was measured in the presence (figure 6a) and in the absence (figure 6b) of TNS.

Results:

The fluorescence of soil washing solution without TNS was not significant and represented only 4.0% of the fluorescence of soil washing solution with TNS.

The soil organic matter does not interfere significantly on Tween 80®-TNS complex fluorescence in the operated diluted ratio.

Using the calibration curve of example 1 or equation (4); the concentration of Tween 80® can be assessed even in the presence of a large quantity of humic substances thanks to the high sensitivity of the method.

Conclusion:

The method according to the present invention has a detection limit of 0.13 mM and a quantification limit of 0.39 mM. The UV absorbance and TOC analysis have demonstrated much lower performance and selectivity than the fluorescence quantification proposed. Such lower performance is due to interference with other organic compounds present in solution (oxidation by-products, phenanthrene). The fluorescence intensity of phenanthrene and by-products are insignificant in this range of concentration (< 3.5%). Soil organic matter has a negligible impact (< 4.0%) due to the operated diluted ratio and the high sensitivity of this method. These results validate the performance of the fluorescence quantification of non-ionic surfactants by using TNS.

**Claims**

1. A method for determining the concentration of non-ionic surfactants in an aqueous solution by excitation-emission fluorescence spectroscopy comprising the steps of:

   (a) contacting a sample of an aqueous solution containing a non-ionic surfactant with 6-*p*-toluidino-2-naphthalenesulfonic acid,
   (b) measuring the emission fluorescence of the sample of said solution,
   (c) deducing the concentration of non-ionic surfactant in the sample from the emission fluorescence intensity.

2. The method according to claim 1 **characterized in that** the concentration of non-ionic surfactant in the sample is deduced from the emission fluorescence intensity using a calibration curve or the equation:

$$F = a \times C_S + b$$

$$\text{with } a = \frac{F_0 V_W K_{mw} k I_0}{k I_0 - K_{mw} F_0 V_W} \text{ and } b = F_0 - \frac{F_0 V_W K_{mw} k I_0}{k I_0 - K_{mw} F_0 V_W} CMC$$

where:

   $K_{mw}$ is the micelle phase/aqueous phase partition coefficient,
   CMC is the critical micelle concentration,
   $C_S$ is the non-ionic surfactant concentration,
   $V_w$ is the molar volume of water,
   F and $F_0$ are the emission fluorescence referred to S and $S_{CMC}$ where S is the apparent solubility of organic compound at surfactant concentration $C_S$, $S_{CMC}$ is the apparent solubility of the organic compound at the CMC,
   k is a constant depending on the equipment, the compounds studied, the molar absorption coefficient and the length of the container, and

$I_0$ is the radiation intensity of excitation.

3. Method according to claim 1 or 2 **characterized in that** the non-ionic surfactant is selected from the group comprising polyethylene glycol tert-octylphenyl ether, polysorbate 20, polysorbate 40, polysorbate 60 and polysorbate 80, polyoxyethylene (20) cetyl ether (Brij 58®), polyoxyethylene (4) lauryl ether (Brij 30®), polyoxyethylene (23) lauryl ether (Brij 35®), octylphenol polyoxyethylene (Igepal CA-720), nonyl phenyl polyoxyethylene glycol (Tergitol NP-10).

4. Method according to anyone of the preceding claims **characterized in that** the concentration of 6-*p*-toluidino-2-naphthalenesulfonic acid in the sample is comprised between $10^{-6}$ M and $10^{-4}$ M.

5. Method according to anyone of the preceding claims **characterized in that** the excitation wavelength is comprised between 200 and 400 nm, preferably between 300 and 350 nm and the emission wavelength is comprised between 300 and 500 nm, preferably between 400 and 450 nm.

6. Method according to anyone of the preceding claims **characterized in that** the surfactant is polysorbate 80.

7. Method according to anyone of claims 1 to 6 **characterized in that** the excitation wavelength is 318 nm and the emission wavelength is 428 nm.

8. Method according to anyone of the preceding claims **characterized in that** the aqueous solutions are selected from soil and groundwater remediation waters, municipal wastewater, industrial wastewater or process water and landfill leachates.

9. Method according to anyone of the preceding claims **characterized in that** the emission fluorescence is measured with a Fluorescence detection device or a High Performance Liquid Chromatography-Fluorescence Detection device.

10. A method for monitoring the concentration of a non-ionic surfactant in a wastewater treatment process comprising the steps of:

(a) determining the concentration of non-ionic surfactant in the treated aqueous solution by excitation-emission fluorescence spectroscopy according to anyone of claims 1 to 9,
(b) comparing the concentration of non-ionic surfactant with the required concentration, and
(c) if necessary, adjusting the concentration of non-ionic surfactant.

11. The method according to claim 10 **characterized in that** the treated aqueous solution is reused in the wastewater treatment process.

12. The method according to claim 10 or 11 **characterized in that** the wastewater treatment process is part of a soil remediation process.

13. A soil remediation process comprising the steps of:

(a) Washing the contaminated soil by contacting an aqueous solution containing a non-ionic surfactant with said soil,
(b) separating the contaminated water from the cleaned soil,
(c) treating the contaminated water,
(d) determining the concentration of non-ionic surfactant in the aqueous solution by excitation-emission fluorescence spectroscopy according to anyone of claims 1 to 9,
(e) if needed, adding non-ionic surfactant, and
(f) recycling the aqueous solution containing the non-ionic surfactant in the soil washing step (a).

14. The process of claim 13 **characterized in that** the treatment in step (c) is electro-Fenton.

15. The use of 6-*p*-toluidino-2-naphthalenesulfonic acid for determining the concentration of non-ionic surfactants in an aqueous solution by excitation-emission fluorescence spectroscopy.

**FIG. 1**

**FIG. 2**

Excitation

FIG. 3A

Excitation

FIG. 3B

FIG. 4A

FIG. 4B

FIG. 5

Excitation

FIG. 6A

Excitation

FIG. 6B

Europäisches
Patentamt

European
Patent Office

Office européen
des brevets

# EUROPEAN SEARCH REPORT

Application Number

EP 13 30 5580

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | HSIN-CHOU CHIANG ET AL: "Interaction of sodium dodecyl sulfate with the hydrophobic fluorescent probe, 2-p-toluidinylnaphthalene-6-sulfonate", THE JOURNAL OF PHYSICAL CHEMISTRY, vol. 79, no. 18, 1 August 1975 (1975-08-01), pages 1935-1939, XP055078678, ISSN: 0022-3654, DOI: 10.1021/j100585a010 | 1,3-15 | INV. G01N33/18 G01N21/64 |
| A | * the whole document * | 2 | |
| X | BEHERA ET AL: "Effect of added ionic liquid on aqueous Triton X-100 micelles", JOURNAL OF COLLOID AND INTERFACE SCIENCE, ACADEMIC PRESS, NEW YORK, NY, US, vol. 307, no. 1, 18 January 2007 (2007-01-18), pages 235-245, XP005899464, ISSN: 0021-9797, DOI: 10.1016/J.JCIS.2006.11.009 | 1,3-15 | |
| A | * page 241, right-hand column * * figures 6,7 * | 2 | **TECHNICAL FIELDS SEARCHED (IPC)** G01N |
| A | EP 0 475 045 A1 (MILES INC [US] BAYER AG [US]) 18 March 1992 (1992-03-18) * paragraphs [0017] - [0024] * | 1-15 | |
| A | US 2005/037509 A1 (GEISLER RICHARD [US] ET AL) 17 February 2005 (2005-02-17) * paragraph [0040] * * claims 1-12 * | 1-15 | |
| A | US 6 045 727 A (AKHAVAN-TAFTI HASHEM [US] ET AL) 4 April 2000 (2000-04-04) * example 39 * | 1-15 | |

The present search report has been drawn up for all claims

| Place of search | Date of completion of the search | Examiner |
|---|---|---|
| Munich | 11 September 2013 | Michalitsch, Richard |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

EP 2 799 866 A1

## ANNEX TO THE EUROPEAN SEARCH REPORT
## ON EUROPEAN PATENT APPLICATION NO.

EP 13 30 5580

This annex lists the patent family members relating to the patent documents cited in the above-mentioned European search report.
The members are as contained in the European Patent Office EDP file on
The European Patent Office is in no way liable for these particulars which are merely given for the purpose of information.

11-09-2013

| Patent document cited in search report | | Publication date | Patent family member(s) | | Publication date |
|---|---|---|---|---|---|
| EP 0475045 | A1 | 18-03-1992 | AU | 628269 B2 | 10-09-1992 |
| | | | AU | 8163491 A | 20-02-1992 |
| | | | CA | 2047966 A1 | 07-02-1992 |
| | | | DE | 69123519 D1 | 23-01-1997 |
| | | | DE | 69123519 T2 | 03-04-1997 |
| | | | EP | 0475045 A1 | 18-03-1992 |
| | | | JP | 3088789 B2 | 18-09-2000 |
| | | | JP | H04250360 A | 07-09-1992 |
| | | | US | 5300439 A | 05-04-1994 |
| US 2005037509 | A1 | 17-02-2005 | NONE | | |
| US 6045727 | A | 04-04-2000 | AT | 236177 T | 15-04-2003 |
| | | | AU | 716233 B2 | 24-02-2000 |
| | | | AU | 1744697 A | 11-08-1997 |
| | | | CA | 2213317 A1 | 24-07-1997 |
| | | | CN | 1180349 A | 29-04-1998 |
| | | | DE | 69720332 D1 | 08-05-2003 |
| | | | DE | 69720332 T2 | 04-12-2003 |
| | | | EP | 0819119 A1 | 21-01-1998 |
| | | | JP | 3169383 B2 | 21-05-2001 |
| | | | JP | H10510848 A | 20-10-1998 |
| | | | JP | 2001158794 A | 12-06-2001 |
| | | | KR | 100259144 B1 | 01-07-2000 |
| | | | US | 6045727 A | 04-04-2000 |
| | | | US | 6045991 A | 04-04-2000 |
| | | | US | 6090571 A | 18-07-2000 |
| | | | US | 6139782 A | 31-10-2000 |
| | | | US | 6218137 B1 | 17-04-2001 |
| | | | US | 6270695 B1 | 07-08-2001 |
| | | | US | 6296787 B1 | 02-10-2001 |
| | | | US | 2003023089 A1 | 30-01-2003 |
| | | | WO | 9726245 A1 | 24-07-1997 |

EPO FORM P0459

For more details about this annex : see Official Journal of the European Patent Office, No. 12/82

18